(19) Europäisches Patentamt
European Patent Office
Office européen des brevets

(11) **EP 2 802 336 B1**

(12) **FASCICULE DE BREVET EUROPEEN**

(45) Date de publication et mention
de la délivrance du brevet:
**03.03.2021 Bulletin 2021/09**

(51) Int Cl.:
*A61K 36/54* (2006.01)   *C11B 1/04* (2006.01)
*C11B 1/06* (2006.01)   *C11B 1/10* (2006.01)
*C11B 3/12* (2006.01)   *A61P 19/02* (2006.01)
*A61P 19/04* (2006.01)

(21) Numéro de dépôt: **13702585.4**

(22) Date de dépôt: **14.01.2013**

(86) Numéro de dépôt international:
**PCT/EP2013/050580**

(87) Numéro de publication internationale:
**WO 2013/104793 (18.07.2013 Gazette 2013/29)**

(54) **UTILISATION DE PEAU D'AVOCAT POUR OBTENIR UN INSAPONIFIABLE D'AVOCAT ENRICHI EN HYDROCARBURES ALIPHATIQUES SATURES ET EN STEROLS**

VERWENDUNG VON AVOCADOSCHALE ZUR HERSTELLUNG VON UNVERSEIFBAREN ANGEREICHERT MIT SATURIERTEN ALIPHATISCHEN HYDROCARBONEN UND MIT STEROLEN

USE OF AVOCADO SKIN TO OBTAIN UNSAPONIFIABLE MATTER ENRICHED WITH SATURATED ALIPHATIC HYDROCARBONS AND WITH STEROLS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **13.01.2012 FR 1250367**

(43) Date de publication de la demande:
**19.11.2014 Bulletin 2014/47**

(73) Titulaire: **Laboratoires Expanscience**
**92048 Paris La Défense Cedex (FR)**

(72) Inventeurs:
• **MSIKA, Philippe**
**F-78000 Versailles (FR)**
• **LEGRAND, Jacques**
**F-61290 Neuilly Sur Eure (FR)**
• **GARNIER, Sébastien**
**06650 Le Rouret (FR)**

(74) Mandataire: **Regimbeau**
**20, rue de Chazelles**
**75847 Paris Cedex 17 (FR)**

(56) Documents cités:
WO-A1-2013/098293   WO-A2-2004/012496
WO-A2-2010/026595   FR-A1- 2 678 632
FR-A1- 2 798 667

• **anonymous: "ASU Expanscience - Cutting Edge Technology to produce a unique and original ASU",**, 13 février 2010 (2010-02-13), pages 1-5, XP002674953, Extrait de l'Internet: URL:http://web.archive.org/web/20100213004 455/http://www.original-asu.com/Cutting_Ed ge__Technology.html [extrait le 2012-04-25]
• **M. J. WERMAN ET AL: "Avocado oil production and chemical characteristics",** JOURNAL OF THE AMERICAN OIL CHEMISTS' SOCIETY, vol. 64, no. 2, 1 février 1987 (1987-02-01), pages 229-232, XP055025527, ISSN: 0003-021X, DOI: 10.1007/BF02542007
• **"Nahrungsfette und Öle - [Extract] ED - BOCKISCH M",** 1 janvier 1993 (1993-01-01), NAHRUNGSFETTE UND -ÖLE, STUTTGART, ULMER, DE, PAGE(S) 73,82,175 - 177, XP009158090, ISBN: 978-3-8001-5817-1 le document en entier
• **KASHMAN Y ET AL: "New compounds from avocado pear",** TETRAHEDRON, ELSEVIER SCIENCE PUBLISHERS, AMSTERDAM, NL, vol. 25, no. 18, 1 janvier 1969 (1969-01-01), pages 4617-4631, XP002090295, ISSN: 0040-4020, DOI: 10.1016/S0040-4020(01)83005-2

- BRIAN I. BROWN: "Isolation of unpleasant flavor compounds in the avocado (Persea americana)", JOURNAL OF AGRICULTURAL AND FOOD CHEMISTRY, vol. 20, no. 4, 1 juillet 1972 (1972-07-01), pages 753-757, XP055025721, ISSN: 0021-8561, DOI: 10.1021/jf60182a019
- GUTFINGER T ET AL: "Studies of unsaponifiables in several vegetable oils", LIPIDS, SPRINGER, US, vol. 9, no. 9, 1 janvier 1974 (1974-01-01) , pages 658-663, XP009158777, ISSN: 0024-4201, DOI: 10.1007/BF02532171
- TZONG-HUEI LEE ET AL: "Heptadecanols from the leaves of Persea americana var. americana", FOOD CHEMISTRY, vol. 132, no. 2, 19 novembre 2011 (2011-11-19), pages 921-924, XP055025945, ISSN: 0308-8146, DOI: 10.1016/j.foodchem.2011.11.067
- Mostert, Mathilda Elizabeth: "Characterization of micro-components of avocado oil extracted with supercritical carbon dioxide and their effect on its oxidative stability", , 15 avril 2008 (2008-04-15), XP002675113, Extrait de l'Internet: URL:http://upetd.up.ac.za/thesis/available /etd-06062008-132406/ [extrait le 2012-05-02]
- BIZIMANA V: "Extraction, characterization, and prediction of the oxidative stability of avocado oil", DISSERTATION, 1997, pages 1-241, XP009158924, University of Minnesota

**Description**

La présente description concerne l'utilisation de peau d'avocat pour obtenir un insaponifiable d'avocat enrichi en hydrocarbures aliphatiques saturés et en stérols. Avantageusement, ladite peau d'avocat représente 5 à 50% en masse par rapport à la masse totale d'avocat utilisé. Les objets de l'invention sont définis dans les revendications. L'invention concerne un procédé d'obtention d'un insaponifiable d'avocat enrichi en hydrocarbures aliphatiques saturés et en stérols à partir d'au moins de la peau d'avocat, ledit insaponifiable contenant au moins 0,2% en masse d'hydrocarbures aliphatiques saturés et au moins 1% en masse de stérols, par rapport à la masse totale de l'insaponifiable, ladite peau d'avocat représentant avantageusement 5 à 50% en masse par rapport à la masse totale d'avocat utilisé, lesdits avocats utilisés n'étant pas des avocats mous ayant une force de résistance à la pénétration dans la chair inférieure ou égale à 3 kg/cm$^2$, ladite force de résistance étant mesurée à l'aide d'un pénétromètre, tel que défini dans la revendication 1. L'invention concerne également un insaponifiable d'avocat enrichi en hydrocarbures aliphatiques saturés et en stérols, susceptible d'être obtenu par ce procédé, caractérisé en ce qu'il contient au moins 0,2 % en masse d'hydrocarbures aliphatiques saturés et au moins 1 % en masse de stérols, par rapport à la masse totale de l'insaponifiable. Enfin, l'invention concerne un tel insaponifiable d'avocat enrichi en hydrocarbures aliphatiques saturés et en stérols, pour son utilisation comme médicament, avantageusement dans la prévention et/ou le traitement des troubles du tissu conjonctif tels que l'arthrose, des pathologies articulaires telles que les rhumatismes, ou encore des maladies parodontales, telles que la gingivite ou la parodontite.

Depuis les années 1990, les débouchés mondiaux de l'avocat se diversifient. Les filières principales restent majoritairement orientées vers l'exportation ou les marchés locaux, en fonction des pays producteurs. Cependant, les volumes destinés à l'industrie sont en forte croissance et sont généralement représentés par la transformation de la pulpe fraîche dans le domaine alimentaire, ainsi que la production d'huile d'avocat de qualité alimentaire ou cosmétique.

Ces dernières applications, si elles restent marginales par rapport aux tonnages dédiés à la commercialisation de l'avocat comme fruit de bouche, permettent de valoriser les écarts de triage et les fruits rejetés par ce marché. Elles représentent une matière première avec un coût d'accès beaucoup plus acceptable.

Ces industries de transformation sont principalement destinées à la valorisation de la pulpe de l'avocat. Elles génèrent de ce fait des sous-produits, résultant du dépulpage des fruits. A titre d'exemple, les filières de production du guacamole et de l'huile d'avocat alimentaire et cosmétique, obtenue par centrifugation, n'utilisent que la pulpe de l'avocat. Ainsi, sont générées des quantités très importantes de coproduits que sont le noyau et la peau du fruit de l'avocat, qui ne sont pas valorisés dans la filière alimentaire.

L'huile d'avocat est en effet concentrée principalement dans les cellules de réserve de la pulpe, i.e. idioblastes. La peau de l'avocat est, quant à elle, pauvre en huile et ne présente donc que peu d'intérêt pour la filière des huileries.

Par ailleurs, dans les procédés classiques d'extraction de l'huile d'avocat, on cherche généralement à éliminer au maximum la peau d'avocat. Pour se faire, les fruits sont ainsi pelés avant extraction de l'huile, car la peau d'avocat contient une haute teneur en chlorophylles et pigments qui colorent et brunissent l'huile finalement obtenue. Ils peuvent également impacter la stabilité oxydative et chimique de l'huile, ainsi que sur son aptitude à être raffinée.

Des débouchés ont été étudiés et développés pour tenter de valoriser ces sous-produits que sont la peau et le noyau : épandage, mulching, horticulture, alimentation animale, mais ils n'apportent pas une forte valeur ajoutée.

Cependant, même si les parties constitutives de l'avocat que sont la peau et le noyau sont naturellement pauvres en huile, elles contiennent des composés constitutifs de l'insaponifiable présentant un potentiel actif intéressant et à forte valeur ajoutée.

Néanmoins, ces composés étant en faibles quantités dans ces parties du fruit, telles que la peau, elles sont de ce fait difficilement accessibles et cxtractiblcs.

La forte teneur en eau des avocats les rend difficiles à travailler et rend presque impossible leur traitement par les procédés physiques d'extraction connus de l'homme du métier et dans des conditions économiques acceptables.

Par ailleurs, la faible teneur en huile et en composés actifs dans la peau d'avocat ne permet pas d'appliquer un traitement physique par pression mécanique, cette technique n'étant pas suffisamment efficace pour traiter des produits présentant des teneurs en huile inférieure à 10%.

Seule l'extraction par solvant apparaît ainsi comme envisageable, mais outre le fait que ce procédé requiert une technologie complexe, il est réputé pour être onéreux, polluant, et toxique pour l'homme et l'environnement.

Il existait donc un besoin de trouver un procédé qui permette, à faible coût, de valoriser ces composés actifs potentiellement disponibles dans des coproduits facilement accessibles.

Durant ces dernières décennies, les connaissances de la chimie de l'avocat se sont considérablement enrichies. Plusieurs familles de composés ont ainsi été isolées et identifiées à partir des fruits et de nombreuses études ont été menées pour mettre en évidence leurs activités biologiques. La composition de la fraction insaponifiable de l'huile d'avocat a été particulièrement étudiée.

Les coproduits de l'industrie alimentaire de l'avocat, et notamment la peau, sont pauvres en huile, mais contiennent tout ou partie des composés constitutifs de l'insaponifiable. En particulier, les composés du type hydrocarbures aliphatiques présentant typiquement un nombre impair d'atomes de carbone, de poids moléculaire voisin de 400 g/mol, et connus pour leurs propriétés protectrices et texturantes.

Par ailleurs, les stérols végétaux ou phytostérols sont également des constituants de l'insaponifiable de l'avocat et sont reconnus entre autres pour leurs propriétés hypocholestérolémiantes, permettant de limiter les risques cardiovasculaires, et pour leurs propriétés anti-inflammatoires.

La demande internationale WO 2013/098293 divulgue un procédé de préparation d'huile d'avocat riche en insaponifiables à partir d'avocats mous entiers, lesdits insaponifiables contenant des acétogénines aliphatiques et/ou leurs dérivés. La demande internationale WO 2013/098293 divulgue ainsi un procédé d'obtention d'une huile d'avocat à partir d'avocats mous entiers, ladite huile contenant au moins 3% en masse insaponifiable par rapport à la masse totale de l'huile, dans laquelle ledit insaponifiable contient des acétogénines aliphatiques et/ou leurs dérivés, comprenant les étapes successives suivantes :

(1) Broyage des avocats mous conduisant à l'obtention d'une granulométrie du broyât comprise entre 2 et 20 mm, en particulier entre 2 et 10 mm,

(2) Séchage du broyât à haute température, avantageusement à une température comprise entre 60 et 150°C, en particulier entre 65 et 120°C, par exemple entre 70 et 100°C, typiquement entre 80 et 100°C, jusqu'à l'obtention d'une humidité résiduelle inférieure ou égale à 5%,

(3) Addition d'eau aux avocats séchés par ajout de 1 à 5 % d'eau ou de vapeur d'eau par rapport à la masse des avocats secs broyés, puis

(4) Extraction de l'huile par pression mécanique.

Le document BIZIMANA V (« Extraction, characterization, and prediction of the oxidative stability of avocado oil », Dissertation, 1997, pages 1-241, University of Minnesota) divulgue des méthodes d'extraction d'huile par solvant, tel que l'hexane, l'éthanol ou l'acétone. En particulier, ce document compare deux méthodes d'extraction par solvant (chloroforme/méthanol versus acétone) des tissus d'avocat.

Il était donc intéressant de trouver un procédé qui permette d'extraire à moindre coût certains composés constitutifs de l'insaponifiable d'avocat, tels que mentionnés ci-dessus, en particulier à partir de co-produits ou sous-produits de la filière alimentaire de l'avocat, tels que la peau d'avocat.

La présente invention vient combler ce besoin. La Demanderesse a ainsi découvert un nouveau procédé d'obtention d'un insaponifiable d'avocat enrichi en hydrocarbures aliphatiques saturés et en stérols, à partir notamment de peau d'avocat.

Le procédé selon la présente invention permet ainsi de valoriser les sous-produits de l'avocat, tels que la peau, et d'extraire avantageusement les composés présents dans la peau qui ne sont pas extractibles par les procédés classiques d'extraction. De tels composés se trouvent solubilisés, puis extraits par l'huile lors de son extraction dans le cadre du procédé objet de la présente invention, et se trouvent de ce fait valorisés.

Par ailleurs, l'augmentation de la proportion de peau permet, de par ses propriétés mécaniques, un échange entre les différentes fractions en contact dans la matière de départ et l'extraction des composés actifs, ce qui a pour conséquence un enrichissement notable de l'huile en composés insaponifiables spécifiques, tels que les stérols et les hydrocarbures aliphatiques saturés.

De façon avantageuse selon la présente invention, l'huile extraite majoritairement de la pulpe joue un rôle de vecteur pour solubiliser et extraire les composés insaponifiables spécifiques contenus dans la peau. De par leur caractère lipophile, ces composés difficilement accessibles au sein de la structure granuleuse de la peau, se trouvent entraînés lors de l'étape d'extraction par pression par le flux d'huile produite qui diffuse dans toute la masse brassée dans le corps de la presse d'extraction.

De façon encore plus avantageuse selon l'invention, il a été mis en évidence le rôle favorable de la température sur le taux d'extraction de ces dérivés lors de l'extraction par pression mécanique de l'huile. Les stérols et hydrocarbures aliphatiques saturés, de par leur structure moléculaire, possèdent un caractère lipophile mais une solubilité limitée dans les corps gras, et de ce fait sont difficilement extractibles. La pression à haute température permet ainsi de réduire l'influence de cette propriété et de garantir un niveau de solubilité suffisant pour obtenir un taux d'extraction performant avec le procédé par pression mécanique.

De façon encore plus particulière selon l'invention, il a été montré que l'augmentation de la proportion de peau par rapport aux différentes parties du fruit dans le mélange de départ devait être très avantageusement accompagnée d'un ajout d'eau ou de vapeur d'eau en amont de la pression pour obtenir un rendement de pression optimal.

Avantageusement selon l'invention, l'apport conjoint de peau dans la masse pour structurer la matrice travaillée dans la presse d'extraction, puis d'eau ou de vapeur d'eau, génère un flux gazeux et une augmentation de pression au sein du produit qui favorise l'éclatement des cellules, la libération de l'huile, sa diffusion dans la masse et son expulsion hors de la cage de la presse d'extraction.

En outre, on peut avantageusement extraire un insaponifiable d'avocat, enrichi en hydrocarbures aliphatiques saturés et en stérols, qui peut être incorporé au sein de compositions cosmétiques, dermatologiques, pharmaceutiques ou de dispositifs médicaux, ou encore dans des compositions alimentaires, des compléments alimentaires ou des nutraceutiques, à visée humaine ou animale.

La présente description divulgue ainsi l'utilisation de peau d'avocat pour obtenir un insaponifiable d'avocat enrichi en hydrocarbures aliphatiques saturés et en stérols, ladite peau d'avocat représentant avantageusement 5 à 50% en masse par rapport à la masse totale d'avocat utilisé. De manière particulièrement avantageuse, la peau d'avocat représente 10 à 40%, typiquement 20 à 40%, en masse par rapport à la masse totale d'avocat utilisé. De manière particulièrement avantageuse, l'insaponifiable selon l'invention contient au moins 0,2% en masse d'hydrocarbures aliphatiques saturés et au moins 1% en masse de stérols, par rapport à la masse totale de l'insaponifiable.

En particulier, la présente description divulgue l'utilisation de peau d'avocat pour obtenir par pression mécanique une huile d'avocat enrichie en hydrocarbures aliphatiques saturés et en stérols, ladite peau d'avocat représentant 5 à 50% ou 10 à 50% en masse par rapport à la masse totale d'avocats utilisés, lesdits hydrocarbures aliphatiques saturés étant typiquement des hydrocarbures à chaîne linéaire non ramifiée présentant un nombre impair de carbones.

Avantageusement, les hydrocarbures aliphatiques saturés sont des alcanes en $C_{27}$, $C_{29}$ ou $C_{31}$, et lesdits stérols sont avantageusement choisis dans le groupe constitué par le β-Sitostérol, le Campestérol, le Stigmastérol, le Δ5-Avenastérol, le Δ7-Stigmastérol, le Citrostadiénol, et leurs mélanges.

La présente description divulgue aussi l'utilisation de l'huile d'avocat contenue dans la pulpe de l'avocat pour extraire les hydrocarbures aliphatiques saturés et les stérols, la pulpe de l'avocat représentant au moins 50% en masse par rapport à la masse totale d'avocat utilisé.

La plupart des variétés d'avocat peuvent être utilisées dans le cadre de la présente invention pour produire de l'huile, puis l'insaponifiable d'avocat, avec les caractéristiques recherchées, dans la mesure où elles renferment le potentiel qualitatif et quantitatif en composés spécifiques.

De manière particulièrement avantageuse, le procédé selon l'invention est appliqué aux variétés les plus cultivées et représentant la *quasi* totalité des tonnages exportés et commercialisés au niveau mondial, préférentiellement les variétés *hass* et *fuerte.*

Dans un mode de réalisation particulier selon la présente invention, les avocats utilisés comme produit de départ sont des avocats entiers, auxquels on peut ajouter avantageusement de la peau. De manière avantageuse, on utilise ainsi comme mélange de départ des avocats entiers auxquels on ajoute de la peau.

Par le terme d'avocats « entiers », on entend, au sens de la présente invention, des avocats contenant la peau, la pulpe et le noyau distribués dans leur intégrité.

Dans un autre mode de réalisation particulier selon la présente invention, les avocats utilisés comme produit de départ sont des avocats sans noyau, auxquels on peut ajouter avantageusement de la peau. De manière avantageuse, on utilise ainsi comme mélange de départ des avocats sans noyau auxquels on ajoute de la peau.

Par le terme d'avocats « sans noyau », on entend, au sens de la présente invention, des avocats entiers dans lesquels le noyau a été retiré, et ne contenant ainsi plus que la peau et la pulpe.

Dans un autre mode de réalisation particulier selon la présente invention, les avocats utilisés comme produit de départ sont de la pulpe d'avocat à laquelle on ajoute de la peau.

Selon une caractéristique particulière de la description, les avocats utilisés sont des avocats mous.

Typiquement, les avocats mous ont un degré de ramollissement équivalent à celui d'une consommation immédiate de l'avocat, et excluent un prétraitement par tranchage.

Avantageusement selon la présente description, les avocats mous sont caractérisés par la consistance de leur chair mesurée à l'aide d'un pénétromètre et définie par une force de résistance à la pénétration. Selon la présente invention, les avocats mous ont une force de résistance à la pénétration dans la chair inférieure ou égale à 3 kg/cm$^2$, typiquement inférieure ou égale à 2 kg/cm$^2$, par exemple inférieure ou égale à 1 kg/cm$^2$.

Selon la présente invention, les avocats utilisés ne sont pas des avocats mous. Plus particulièrement, selon un mode de réalisation de l'invention, les avocats utilisés sont des avocats durs.

Selon la présente invention, les avocats durs ont une force de résistance à la pénétration dans la chair supérieure à 3 kg/cm$^2$.

Typiquement selon la présente invention, la force de pénétration est mesurée à l'aide d'un pénétromètre du type PCE-PTR 200 ou FT 327, qui mesure la force en kilogramme nécessaire pour faire pénétrer un embout calibré dans le fruit. Avantageusement, le fruit est pelé avant de faire la mesure afin de s'affranchir de la résistance de la peau (tégument) et de la variabilité des différentes variétés d'avocat testées. La tige (pointal ou embout) utilisée pour cette mesure a un diamètre nominal respectivement de 6 et 11,3 mm.

Selon l'invention, les avocats utilisés sont broyés, puis séchés à haute température, entre 60 et 150°C, jusqu'à l'obtention d'une humidité résiduelle inférieure ou égale à 5%, avant l'obtention de l'huile par pression mécanique.

Selon la présente invention, suite au broyage et au séchage des avocats, 1 à 5 % d'eau ou de vapeur d'eau, par rapport à la masse des avocats secs, est additionné, avant l'obtention de l'huile par pression mécanique.

Il a en effet été découvert que lorsque l'on intègre une étape d'injection d'eau ou de vapeur d'eau au sein des avocats séchés, ceci permet d'obtenir une huile d'avocat enrichie en hydrocarbures aliphatiques saturés et en stérols avec un rendement élevé.

La présente description divulgue ainsi l'utilisation de peau d'avocat pour obtenir un insaponifiable d'avocat enrichi en hydrocarbures aliphatiques saturés et en stérols. L'insaponifiable d'un corps gras comprend l'ensemble des constituants de ce corps gras qui, après saponification en milieu fortement alcalin, sont très peu solubles ou insolubles dans l'eau, et solubles dans des solvants organiques, tels que l'éther éthylique, les hydrocarbures aromatiques, les solvants chlorés....

L'insaponifiable est donc composé de tous les constituants non hydrolysables du corps gras, ainsi que ceux résultant majoritairement de la saponification d'esters non glycéridiques d'acides gras (esters de stérols, cires, esters de tocophérols...).

Quatre grands groupes ou familles de substances sont généralement présents dans la plupart des insaponifiables d'huiles végétales. Le groupe le plus important en masse est représenté par celui qui rassemble les stérols, les alcools triterpéniques pentacycliques et les 4-méthylstérols. Le second groupe est constitué par les tocophérols pouvant intégrer des tocotriènols. Les deux autres groupes sont les alcools aliphatiques et les hydrocarbures aliphatiques saturés et insaturés.

L'insaponifiable d'avocat selon l'invention contient des hydrocarbures aliphatiques saturés et des stérols, et est enrichi en hydrocarbures aliphatiques saturés et en stérols.

Par le terme d'insaponifiable « enrichi en hydrocarbures aliphatiques saturés », on entend au sens de la présente invention un insaponifiable contenant au moins 0,2% en masse d'hydrocarbures aliphatiques saturés, par rapport à la masse totale de l'insaponifiable.

Avantageusement, l'insaponifiable selon l'invention contient entre 0,2 et 10% en masse, plus avantageusement entre 0,3 et 5% en masse, en particulier entre 0,4 et 2% en masse, d'hydrocarbures aliphatiques saturés par rapport à la masse totale de l'insaponifiable.

Dans le cadre de l'invention, les hydrocarbures aliphatiques saturés sont notamment des hydrocarbures à chaîne linéaire non ramifiée présentant un nombre impair de carbones. Avantageusement, les hydrocarbures aliphatiques saturés sont des alcanes en $C_{27}$, $C_{29}$ ou $C_{31}$.

Typiquement, les hydrocarbures aliphatiques saturés selon l'invention sont choisis dans le groupe constitué par le n-heptacosane ($CH_3(CH_2)_{25}CH_3$), le n-nonacosanc ($CH_3(CH_2)_{27}CH_3$), le n-hcntriacontanc ($CH_3(CH_2)_{29}CH_3$), et leurs mélanges.

Par le terme d'insaponifiable « enrichi en stérols », on entend au sens de la présente invention un insaponifiable contenant au moins 1% en masse de stérols, par rapport à la masse totale de l'insaponifiable.

Avantageusement, l'insaponifiable selon l'invention contient entre 1 et 20% en masse de stérols, plus avantageusement entre 2 et 18% en masse, en particulier entre 3 et 15% en masse, de stérols par rapport à la masse totale de l'insaponifiable.

Les stérols sont notamment des hydrocarbures tétracycliques comportant une fonction alcool en position 3 et une double liaison dont la position intracyclique est majoritairement en position 5.

Dans le cadre de l'invention, les stérols sont avantageusement choisis dans le groupe constitué par le β-Sitostérol, le Campestérol, le Stigmastérol, le Δ5-Avenastérol, le Δ7-Stigmastérol, le Citrostadiénol, et leurs mélanges.

Selon la présente invention, l'insaponifiable contient au moins 0,2% en masse, avantageusement entre 0,2 et 10% en masse, typiquement entre 0,3 et 5% en masse, d'hydrocarbures aliphatiques saturés, par rapport à la masse totale de l'insaponifiable, et contient au moins 1% en masse, avantageusement entre 1 et 20% en masse, typiquement entre 2 et 18% en masse, de stérols, par rapport à la masse totale de l'insaponifiable.

La composition de l'insaponifiable d'avocat selon la présente invention se différentie de la composition classiquement trouvée dans les huiles végétales, car elle intègre majoritairement des constituants spécifiques de l'avocat. En particulier, la fraction majoritaire de l'insaponifiable d'avocat est représentée par le groupe des furanes aliphatiques. La deuxième famille de molécules regroupe les alcools gras polyhydroxylés. La troisième famille est constituée par les stérols incluant les alcools triterpéniques pentacycliques et les 4-méthylstérols. Les autres familles intègrent notamment les hydrocarbures aliphatiques saturés.

Typiquement, l'insaponifiable de l'huile selon l'invention contient également des alkyls furanes et/ou des alkyls polyols.

Les alkyls furanes ou furanes aliphatiques sont encore appelés lipides furaniques ou plus communément avocadofuranes. Il s'agit notamment de dérivés des persins comportant un groupement furane, qui résultent en particulier de la transformation chimique par déshydratation et cyclisation intramoléculaire des persins extraites de l'avocat. A titre d'exemple, on peut citer les 2-alkyls furanes.

Avantageusement, l'insaponifiable selon l'invention contient entre 30 et 70% en masse d'alkyls furanes, par rapport à la masse totale de l'insaponifiable.

Les alkyls polyols ou alcools gras polyhydroxylés sont notamment des triols du type 1,2,4-trihydroxy à longues chaînes saturées ou poly-insaturées, éthyléniques ou acétyléniques.

Avantageusement, l'insaponifiable selon l'invention contient entre 5 et 30% en masse d'alkyls polyols, par rapport à la masse totale de l'insaponifiable.

La présente description divulgue un procédé d'obtention d'un insaponifiable d'avocat enrichi en hydrocarbures aliphatiques saturés et en stérols à partir d'au moins de la peau d'avocat, ladite peau d'avocat représentant 5 à 50% en masse par rapport à la masse totale d'avocat utilisé, comprenant les étapes successives suivantes :

- Tranchage ou broyage d'avocats contenant une teneur en peau de 5 à 50% m/m,
- Séchage à haute température, avantageusement à une température comprise entre 60 et 150°C, en particulier entre 80 et 120°C,
- Extraction de l'huile,
- alternativement :

    - a. Traitement thermique de l'huile extraite à une température avantageusement comprise entre 80 et 150°C, puis concentration de l'huile en sa fraction insaponifiable, ou bien
    - b. Concentration de l'huile en sa fraction insaponifiable, puis traitement thermique à une température avantageusement comprise entre 80 et 150°C,

- suivie d'une étape de saponification et d'extraction de l'insaponifiable,
- et éventuellement au moins une purification et/ou un fractionnement.

La présente invention a pour objet un procédé d'obtention d'un insaponifiable d'avocat enrichi en hydrocarbures aliphatiques saturés et en stérols à partir d'au moins de la peau d'avocat, ledit insaponifiable contenant au moins 0,2% en masse d'hydrocarbures aliphatiques saturés et au moins 1% en masse de stérols, par rapport à la masse totale de l'insaponifiable, ladite peau d'avocat représentant 5 à 50% en masse par rapport à la masse totale d'avocat utilisé, lesdits avocats utilisés n'étant pas des avocats mous ayant une force de résistance à la pénétration dans la chair inférieure ou égale à 3 kg/cm$^2$, ladite force de résistance étant mesurée à l'aide d'un pénétromètre, et ledit procédé comprenant les étapes successives suivantes :

(1) Tranchage ou broyage d'avocats, lesdits avocats contenant une teneur en peau de 5 à 50% m/m, ledit broyage conduisant à l'obtention d'une granulométrie de broyat comprise entre 2 et 20 mm,

(2) Séchage à haute température comprise entre 60 et 150°C, en particulier entre 80 et 120°C, jusqu'à obtention d'une humidité résiduelle inférieure ou égale à 5%.

(3) Addition d'eau aux avocats séchés par ajout de 1 à 5% d'eau ou de vapeur d'eau par rapport à la masse des avocats secs, puis avantageusement homogénéisation par malaxage, avant introduction dans la presse, puis

(4) Extraction de l'huile par pression mécanique, avantageusement à une température comprise entre 80 et 100°C, puis

(5) alternativement :

    - a. Traitement thermique de l'huile extraite à une température comprise entre 80 et 150°C, puis concentration de l'huile en sa fraction insaponifiable, ou bien
    - b. Concentration de l'huile en sa fraction insaponifiable, puis traitement thermique à une température comprise entre 80 et 150°C,

(6) suivie d'une étape de saponification et d'extraction de l'insaponifiable,

(7) et éventuellement au moins une étape de purification et/ou de fractionnement.

Selon la présente invention, l'insaponifiable contient au moins 0,2% en masse d'hydrocarbures aliphatiques saturés et au moins 1% en masse de stérols, par rapport à la masse totale de l'insaponifiable.

Dans un mode de réalisation particulier du procédé selon la présente invention, les avocats utilisés comme produit de départ sont des avocats entiers, auxquels on peut ajouter avantageusement de la peau. De manière avantageuse, on utilise ainsi comme mélange de départ des avocats entiers auxquels on ajoute de la peau.

Les avocats entiers sont tels que définis ci-dessus.

Dans un autre mode de réalisation particulier du procédé selon la présente invention, les avocats utilisés comme produit de départ sont des avocats sans noyau, auxquels on peut ajouter avantageusement de la peau. De manière avantageuse, on utilise ainsi comme mélange de départ des avocats sans noyau auxquels on ajoute de la peau.

Les avocats sans noyau sont tels que définis ci-dessus.

Dans un autre mode de réalisation particulier du procédé selon la présente invention, les avocats utilisés comme produit de départ sont de la pulpe d'avocat à laquelle on ajoute de la peau.

Avantageusement selon l'invention, les avocats utilisés comme produit de départ ont une teneur en peau d'avocat comprise entre 10 et 40% en masse, typiquement 20 à 40%, par rapport à la masse totale d'avocat utilisé.

Dans un mode de réalisation particulier selon la présente invention, le broyage (1) est réalisé sur les avocats entiers constitués de la peau, de la pulpe et du noyau, ou encore sur les avocats sans noyau constitués de la peau et de la pulpe.

Le broyage (1) permet avantageusement de déchiqueter la peau, de morceler le noyau et de malaxer le mélange afin d'obtenir une dispersion et une granulométrie homogènes du broyat (particules et morceaux obtenus) dans la pulpe d'avocat.

Typiquement, les broyeurs utilisés s'adaptent à la très grande différence de texture et de dureté des différentes parties qui composent l'avocat : peau, pulpe et noyau. Ainsi, la technologie de broyeurs à mettre en œuvre doit permettre de traiter des matériaux présentant des parties très dures (noyau) et des parties plus tendres (peau), voire très molles (pulpe).

Le broyage (1) est avantageusement réalisé à l'aide d'un broyeur de type à couteaux ou à rouleaux dentelés.

La configuration et le réglage doivent cependant être généralement adaptés en fonction de la taille des fruits, de leur maturité et de leur qualité (biométrie noyau, pulpe et peau) pour aboutir à la granulométrie avantageusement recherchée.

Le broyage (1) est réalisé de telle manière à obtenir une taille spécifique des particules et morceaux, répondant à une fourchette de granulométrie qui donne au broyat une texture adaptée à un séchage rapide. Cette texture se caractérise par un aspect visuel non continu, les morceaux étant détectables généralement dans la masse sans utilisation d'un instrument optique complémentaire. Typiquement, la surface du broyat n'est pas lisse et comporte des aspérités représentées par les particules de noyau et de peau. Lors du séchage, le broyat ne forme pas une masse compacte, mais des blocs facilement friables.

Selon la présente invention, le broyage (1) conduit à l'obtention d'une granulométrie du broyat comprise entre 2 et 20 mm, en particulier entre 2 et 10 mm.

De manière avantageuse selon l'invention, le mélange, une fois broyé, doit être réparti de façon adaptée pour garantir une homogénéité du séchage (2) qui suit avec la plus grande efficacité, avantageusement par étalement en couche mince, typiquement pour conduire à un film de faible épaisseur avantageusement comprise entre 0,5 et 5 cm, en particulier entre 1 et 2 cm, ou encore par mise en forme permettant d'optimiser la surface d'évaporation du type extrusion ou passage dans une filière.

Selon une autre caractéristique de l'invention, les avocats utilisés sont des avocats durs. Les avocats durs sont tels que définis ci-dessus.

Dans ce cas, la première étape du procédé selon l'invention consiste en un tranchage des avocats durs.

Le tranchage (1) des avocats est typiquement opéré à l'aide d'une trancheuse à disque.

Le tranchage (1) des avocats conduit avantageusement à des tranches d'épaisseur de 2 à 5 mm.

Les tranches sont ensuite réparties de façon homogène sur des plateaux ou claies de séchage.

L'étape suivante de séchage (2) a pour but d'extraire l'eau du milieu, mais aussi de rendre extractibles les composés de l'insaponifiable. Elle est réalisée, en particulier, grâce à une technologie spécifique et à une température choisie pour optimiser les besoins en énergie et limiter les réactions indésirables. En effet, une température trop basse limiterait la vitesse d'évaporation de l'eau et favoriserait l'action des lipases, entraînant l'hydrolyse des glycérides et l'augmentation de l'indice d'acide du milieu. Une température trop élevée favoriserait le phénomène de croûtage, ainsi que la dégradation thermique et/ou oxydative ou non (réaction de Maillard) des composés sensibles de l'insaponifiable ou des composés insaturés de l'huile.

Il a ainsi été découvert qu'il était recommandé d'utiliser une étape de séchage à température ménagée et contrôlée. La teneur en eau très élevée de l'avocat (≈ 75%) requiert ainsi une technique de séchage très efficace et spécifique pour garantir une évaporation rapide n'induisant pas de dégradation des constituants propres du fruit.

Selon l'invention, le séchage (2) est réalisé à température ménagée et contrôlée à une température comprise entre 60 et 150°C, en particulier entre 65 et 120°C, par exemple entre 80 et 120°C ou entre 70 et 100°C, typiquement entre 80 et 100°C.

Selon une caractéristique particulière de l'invention, le séchage (2) est réalisé pendant 8 à 78 h, avantageusement pendant 10 à 24 h.

Le séchage (2) selon l'invention peut être réalisé en particulier par séchage sous courant d'air chaud ou sous atmosphère contrôlée (ex. azote), par séchage à pression atmosphérique ou sous vide, ou par séchage par micro-ondes.

Dans le cadre du présent procédé, pour des raisons de facilité de mise en œuvre industrielle et pour des raisons de coût, le séchage en séchoirs ventilés, en couche mince et sous courant d'air chaud, à une température comprise entre 80 et 100°C, pendant 8 à 36 heures est préféré.

A l'issue du séchage (2), le produit séché présente un taux d'humidité résiduelle inférieure ou égale à 5% m/m.

L'humidité résiduelle est typiquement mesurée à l'aide d'une méthode thermogravimétrique par séchage IR. D'autres méthodes peuvent également être mises en œuvre, telles que la méthode de perte au séchage en étuve ou la méthode de titrage Karl Fischer.

Une humidité résiduelle inférieure ou égale à 5% joue un rôle important dans la consistance des avocats séchés, leur conférant une texture solide cassante favorable pour résister aux contraintes physiques développées pendant la pression mécanique. Au-delà de 5% d'humidité, l'avocat séché présente une consistance molle qui conduit, lors de la pression, à la formation d'une purée sans consistance suffisante pour être pressée efficacement.

Il a été trouvé que, même avec une humidité résiduelle inférieure ou égale à 5 % et une consistance propice à la pression, ces conditions ne sont pas totalement suffisantes pour atteindre des conditions de pression permettant un rendement d'extraction de l'huile élevé.

Il a ainsi été découvert de façon surprenante qu'un réajustement (3) de l'humidité résiduelle par addition de 1 à 5% d'eau ou de vapeur d'eau, par exemple de 1 à 3 % d'eau ou de vapeur d'eau, par rapport à la masse des avocats secs, permettait d'accroitre considérablement le taux d'extraction de l'huile, et de ce fait l'efficacité de la pression.

Cette opération de réajustement/addition d'eau (3) doit être réalisée juste avant l'introduction des fruits séchés dans la presse par addition d'eau purifiée ou de vapeur d'eau pour que les avocats secs se saturent en humidité sans perdre leur consistance ferme et afin qu'aucun ramollissement n'apparaisse.

Ce taux d'humidité résiduelle pour être efficace ne peut être obtenu que par déshydratation préalable (2) des avocats, puis réajustement par addition d'eau (3). En effet, une déshydratation partielle contrôlée directe n'aboutit pas à une texture de produit compatible avec la pression, comme il a été signalé précédemment.

De façon particulière selon l'invention, l'addition d'eau (3) dans les avocats séchés est réalisée par ajout contrôlé d'eau ou de vapeur d'eau sur les avocats séchés, puis homogénéisation par malaxage typiquement dans un mélangeur planétaire, avantageusement pendant 1/2h à 1h.

De façon plus particulière selon l'invention, l'addition d'eau (3) dans les avocats séchés est réalisée en continu dans un convoyeur du type vis sans fin. L'eau ou la vapeur d'eau est ajoutée sur les avocats en tête de convoyeur et l'homogénéisation est obtenue par agitation dans le convoyeur, lors du déplacement du produit. Le dimensionnement du convoyeur doit permettre de garantir typiquement un temps de séjour minimum de 1/2h des avocats séchés.

De façon encore plus particulière selon l'invention, le convoyeur est utilisé pour alimenter la presse d'extraction.

L'étape d'extraction (4) de l'huile est ainsi précédée d'une étape d'addition d'eau (3) dans les avocats séchés par ajout de 1 à 5%, de préférence de 1 à 3% d'eau ou de vapeur d'eau, par rapport à la masse des avocats séchés.

L'étape d'extraction (4) de l'huile est mise en œuvre par une pression mécanique de la matière sèche, après mise en œuvre de l'étape d'addition d'eau (3).

Usuellement, pour pouvoir travailler avec efficacité, les presses d'extraction doivent recevoir une matière contenant une teneur en fibres et matière organique adaptée conférant au tourteau produit une certaine consistance. Cette consistance permet d'atteindre, en tête de presse, une pression élevée indispensable pour garantir un rendement de pression adapté.

Avantageusement, la présence de particules de peau à l'intérieur du mélange lui donne une consistance et une texture favorable à l'extraction de l'huile par pression et améliore de ce fait la productivité et la production de l'huile par pression.

Avantageusement, l'étape d'extraction (4) est réalisée à une température comprise entre 80 et 100°C. Cette température est obtenue et maintenue constante par tous les moyens à disposition tels que, préchauffage du corps du presse, préchauffage des avocats séchés puis additionnés d'eau en alimentation de la presse, chauffage du corps de presse...

En particulier, le maintien de la température de travail de la presse à une valeur supérieure à 80°C, plus particulièrement à 100°C, permet de garantir un bon niveau d'extraction de l'huile et d'améliorer notablement le rendement d'extraction des constituants de l'insaponifiable, notamment les stérols et/ou les hydrocarbures aliphatiques saturés.

L'étape d'extraction (4) selon l'invention est généralement complétée par une filtration qui élimine les particules solides et garantit la limpidité de l'huile produite.

L'étape de concentration de l'huile en sa fraction insaponifiable pour préparer un concentrat, mise en œuvre à l'étape (5)a. ou (5)b., est en particulier décrite ci-dessous.

L'étape de concentration de l'huile en sa fraction insaponifiable (5)a. ou (5)b. est généralement opérée par cristallisation à froid ou par distillation moléculaire. Avantageusement, le concentrat en insaponifiable d'huile d'avocat est préparé par distillation moléculaire, typiquement à une température comprise entre 180 et 260°C, en maintenant une pression comprise entre $10^{-2}$ et $10^{-3}$ mmHg.

Cette étape de distillation moléculaire de l'huile d'avocat est de préférence réalisée en utilisant un dispositif choisi parmi les distillateurs moléculaires de type centrifuge et les dispositifs de type film raclé.

L'étape de traitement thermique de l'huile ou du concentrat, mise en œuvre à l'étape (5)a. ou (5)b., est réalisée à une température comprise entre 80 et 150°C, par exemple entre 80 et 130°C.

Le procédé de préparation de l'insaponifiable d'avocat enrichi en hydrocarbures aliphatiques saturés et en stérols comprend une étape (6) de saponification et d'extraction de l'insaponifiable, par exemple à l'aide d'un solvant.

En particulier, l'étape (6) de saponification et d'extraction de l'insaponifiable peut être mise en œuvre en présence de potasse ou de soude en milieu alcoolique, de préférence éthanolique, suivie d'une ou plusieurs extraction(s). L'extraction par un solvant organique approprié (extraction liquide-liquide) en vue de séparer les savons d'acides gras et les composés insaponifiables, est particulièrement adaptée. Le solvant organique approprié peut être, par exemple choisi dans le groupe des alcanes, des alcanes halogénés, des solvants aromatiques et aromatiques halogénés, des éthers, des cétones, des esters, des solvants comprenant au moins un atome de silicium ou tout autre solvant approprié non miscible avec la solution hydro-alcoolique.

Suite à l'extraction de l'insaponifiable, on peut procéder à des étapes complémentaires (7) de purification ou de fractionnement.

La présente invention a également pour objet un insaponifiable d'avocat enrichi en hydrocarbures aliphatiques saturés et en stérols, susceptible d'être obtenu par le procédé selon l'invention.

L'insaponifiable selon l'invention contient au moins 0,2% en masse, avantageusement entre 0,2 et 10% en masse, plus avantageusement entre 0,3 et 5% en masse, en particulier entre 0,4 et 2% en masse, d'hydrocarbures aliphatiques saturés, par rapport à la masse totale de l'insaponifiable.

Les hydrocarbures aliphatiques saturés sont tels que définis précédemment et sont typiquement des hydrocarbures à chaîne linéaire non ramifiée présentant un nombre impair de carbones. Avantageusement, les hydrocarbures aliphatiques saturés sont des alcanes en $C_{27}$, $C_{29}$ ou $C_{31}$, en particulier choisis dans le groupe constitué par le n-heptacosane, le n-nonacosane, le n-hentriacontane, et leurs mélanges.

L'insaponifiable selon l'invention contient au moins 1% en masse, avantageusement entre 1 et 20% en masse, plus avantageusement entre 2 et 18% en masse, en particulier entre 3 et 15% en masse de stérols, par rapport à la masse totale de l'insaponifiable.

Les stérols sont tels que définis précédemment et sont typiquement choisis dans le groupe constitué par le β-Sitostérol, le Campestérol, le Stigmastérol, le Δ5-Avenastérol, le Δ7-Stigmastérol, le Citrostadiénol, et leurs mélanges.

Selon la présente invention, l'insaponifiable contient au moins 0,2% en masse, avantageusement entre 0,2 et 10% en masse, typiquement entre 0,3 et 5% en masse, d'hydrocarbures aliphatiques saturés, par rapport à la masse totale de l'insaponifiable, et contient au moins 1% en masse, avantageusement entre 1 et 20% en masse, typiquement entre 2 et 18% en masse, de stérols, par rapport à la masse totale de l'insaponifiable.

Typiquement, l'insaponifiable de l'huile selon l'invention contient également des alkyls furanes et/ou des alkyls polyols, tels que définis ci-dessus.

Avantageusement, l'insaponifiable selon l'invention contient entre 30 et 70% en masse d'alkyls furanes, par rapport à la masse totale de l'insaponifiable.

Par ailleurs, l'insaponifiable selon l'invention contient généralement entre 5 et 30% en masse d'alkyls polyols, par rapport à la masse totale de l'insaponifiable.

La présente description divulgue également une composition contenant un insaponifiable selon l'invention tel que mentionné ci-dessus, avantageusement à une concentration comprise entre 0,1 et 99,9% en masse, encore plus avantageusement de 30 à 70% en masse, par rapport à la masse totale de la composition.

Ladite composition peut, en outre, comprendre d'autres actifs.

Parmi les actifs recommandés en association avec l'insaponifiable selon l'invention, on peut citer les extraits végétaux, en particulier :

- Les huiles ou beurres végétaux tels que les huiles de Soja et/ou l'huile de Colza, l'huile de Lupin, avantageusement l'huile de Lupin blanc doux, ou un mélange de ces huiles ou beurres ;
- l'oléodistillat ou les concentrats d'huile végétale ou animale, notamment de tournesol, plus avantageusement des concentrats de Tournesol linoléiques, tels que l'huile de tournesol concentrée en insaponifiable (Soline®) commercialisée par les Laboratoires Expanscience, les huiles concentrées en insaponifiable du type huile de soja, de colza, de maïs ou de palme ;
- les insaponifiables de végétaux ou d'huile végétale, avantageusement des furanes d'avocat (Avocadofurane®), les insaponifiables d'Avocat et/ou de Soja, plus particulièrement un mélange d'insaponifiables d'Avocat furaniques et d'insaponifiables de Soja, avantageusement dans un rapport respectif d'environ 1/3-2/3 (tel que Piasclédine®300), les insaponifiables de soja, les insaponifiables stéroliques (typiquement des insaponifiables dont la teneur globale en stérols, en méthylstérols et en alcools triterpèniques, est comprise entre 20 et 95 % en masse, de préférence 45-65 % en masse, par rapport à la masse totale de l'insaponifiable), les phytostérols, les esters de stérols et les dérivés vitaminiques.

En particulier, la composition contient un insaponifiable d'avocat selon l'invention, en association avec un insaponifiable de soja, avantageusement dans un rapport d'environ 2/3 pour le soja et 1/3 pour l'avocat (tel que Piasclédine®300).

Dans un mode de réalisation particulier, ladite composition comprend en outre au moins un composé choisi parmi les composés suivants, connus pour leurs propriétés dans le traitement du tissu conjonctif, en particulier dans le traitement de l'arthrose :

les sucres aminés tels que la glucosamine ; les sels de glucosamine, tels que le chlorhydrate de glucosamine (par exemple de 1500 à 2000 mg/j), le sulfate de glucosamine, le phosphate de glucosamine et le N-acétylglucosamine; les glycosaminoglycanes (GAG) tels que la chondroïtine sulfate (par exemple de 800 à 1200 mg/j) ; les analogues de glycosaminoglycanes tels que les glycosaminoglycanes polysulfatés, ou les précurseurs de glycosaminoglycanes tels que l'acide hyaluronique, l'acide glucuronique, l'acide iduronique, le keratane sulfate, le heparane sulfate, ou le dermatine sulfate ; la pentosane ou ses dérivés, en particulier la pentosane sulfate, la pentosane polysulfate (PPS) et les polysaccharides pentosane polysulfates; la S-adénosylméthionine (SAMe) ; l'adénosine ; la superoxyde dismutase (SOD) ; la L-ergothionine ; les collagènes de type II hydrolysés ou non; les hydrolysats de collagène tels que la gélatine ; la diacérine ; l'acide arachidonique ; la tétracycline ; les composés analogues de la tétracycline ; la doxycycline ; l'hydroxyproline ; et leurs mélanges.

Avantageusement, ladite composition comprend en association plusieurs des composés mentionnés ci-dessus. La glucosamine et la chondroïtine sulfate, seuls ou en association, sont des composés particulièrement préférés.

Dans un autre mode de réalisation particulier, la composition comprend en outre au moins un anti-inflammatoire non stéroïdien (AINS), tel que le paracétamol.

Enfin, la présente invention a également pour objet l'insaponifiable d'avocat enrichi en hydrocarbures aliphatiques saturés et en stérols tel que décrit ci-dessus, pour son utilisation comme médicament, , à visée humaine ou animale, avantageusement dans la prévention et/ou le traitement des troubles du tissu conjonctif tels que l'arthrose, des pathologies articulaires telles que les rhumatismes, des maladies parodontales, telles que la gingivite ou la parodontite.

La présente description divulgue également l'insaponifiable d'avocat enrichi en hydrocarbures aliphatiques saturés et en stérols tel que décrit ci-dessus pour son utilisation comme médicament dans la prévention et/ou le traitement des troubles du derme et/ou de l'hypoderme tels que le vieillissement cutané, les vergetures et la cellulite, ou encore des troubles de la barrière épidermique tels que les inflammations cutanées, l'eczéma atopique et les dermatites irritatives et/ou inflammatoires. Il est également décrit la composition telle que décrite ci-dessus, pour son utilisation comme médicament, ainsi que l'insaponifiable d'avocat enrichi en hydrocarbures aliphatiques saturés et en stérols tel que décrit ci-dessus ou la composition telle que décrite ci-dessus, pour son utilisation comme dispositif médical, comme agent dermatologique, comme agent cosmétique, ou comme nutraceutique, à visée humaine ou animale, avantageusement dans la prévention et/ou le traitement des troubles du tissu conjonctif tels que l'arthrose, des pathologies articulaires telles que les rhumatismes, des maladies parodontales, telles que la gingivite ou la parodontite, ou encore dans la prévention et/ou le traitement des troubles du derme et/ou de l'hypoderme tels que le vieillissement cutané, les vergetures et la cellulite, ou encore des troubles de la barrière épidermique tels que les inflammations cutanées, l'eczéma atopique et les dermatites irritatives et/ou inflammatoires.

La présente invention a pour objet un insaponifiable d'avocat enrichi en hydrocarbures aliphatiques saturés et en stérols, susceptible d'être obtenu selon le procédé de l'invention, pour son utilisation comme médicament, avantageusement dans la prévention et/ou le traitement des troubles du tissu conjonctif tels que l'arthrose, des pathologies articulaires telles que les rhumatismes, ou encore des maladies parodontales, telles que la gingivite ou la parodontite.

Par ailleurs, on entend par dispositif médical selon l'invention tout instrument, appareil, équipement, matière, produit, à l'exception des produits d'origine humaine, ou autre article seul ou en association, destiné par le fabricant à être utilisé chez l'homme à des fins médicales et dont l'action principale voulue n'est pas obtenue par des moyens pharmacologiques ou immunologiques ni par métabolisme, mais dont la fonction peut être assistée par de tels moyens.

Avantageusement, les compositions divulguées dans la présente description sont adaptées à une administration orale, telles qu'une composition pharmaceutique ou un médicament, un complément alimentaire ou une composition nutraceutique.

Selon une variante, les compositions divulguées dans la présente description sont adaptées à l'administration topique et incluent notamment les crèmes, les émulsions, les laits, les pommades, les lotions, les huiles, les solutions aqueuses ou hydro-alcooliques ou glycoliques, les poudres, les patchs, les sprays, les shampooings, les vernis ou tout autre produit pour application externe.

Les exemples suivants sont donnés pour illustrer l'invention :

Exemple 1 : apport de l'addition d'eau dans les avocats séchés avant pression et de la température de pression.

3 kg d'avocats d'origine kényane, de calibre 18, de variété *hass* et de dureté mesurée à l'aide d'un pénétromètre, supérieure à 13 Kg/cm$^2$ et dont la part de peau représente 14,3% en masse, sont broyés dans un broyeur à couteaux RETSCH du type SM 100 équipé selon la granulométrie souhaitée de tamis de fond à ouverture de maille de 10 mm.

Le broyat est déposé sur un plateau de 0,158 m$^2$, sur une épaisseur de 2 cm.

Le plateau est ensuite disposé dans une étuve ventilée et le séchage est réalisé sous une circulation d'air à 80 ± 5 °C pendant 24 heures.

Le broyat séché est récupéré et broyé pour homogénéisation sur le broyeur à couteaux RETSCH du type SM 100 équipé de tamis de fond à ouverture de maille de 20 mm.

Suivant les essais, l'eau est ensuite ajoutée ou non sur le broyat par vaporisation d'une quantité d'eau purifiée adaptée et le mélange est homogénéisé dans un mélangeur planétaire juste avant d'être introduit dans la presse pour extraction de l'huile.

L'huile d'avocat est ensuite extraite par pression mécanique sur une presse à vis de laboratoire du type Komet préchauffée à la température déterminée de l'essai.

Les quantités d'huile et de tourteau sont déterminées par pesée pour chaque essai et le rendement d'extraction est calculé par la formule suivante :

$$\text{Masse d'huile récupérée} \times 100 / (\text{Masse d'huile récupérée} + \text{masse de tourteau}).$$

Les huiles d'avocat correspondant aux différents essais sont filtrées, puis distillées sur un distillateur moléculaire à film raclé du type Leybold KDL 4 à une température de 230°C et sous un vide de $10^{-2}$ à $10^{-3}$ mmHg.

Les distillats concentrés en insaponifiable sont chauffés à 85°C pendant 48h sous courant d'azote dans un réacteur agité.

Les distillats sont ensuite mis en solution dans l'alcool éthylique et saponifiés avec de la potasse à 50% et par chauffage à reflux de l'alcool pendant 4 heures.

Les mélanges réactionnels sont alors dilués par de l'eau purifiée, puis extraits avec du 1,2-dichloroéthane en ampoule à décantée. L'extraction est poursuivie avec des volumes répétés de solvant jusqu'à obtention de phases organiques inférieures incolores.

Les phases organiques sont regroupées, lavées à l'eau jusqu'à pH neutre, séchées sur sulfate de sodium anhydre puis évaporées sous vide dans un évaporateur rotatif pour éliminer le solvant et récupérer l'insaponifiable. Les produits récupérés sont alors conditionnés sous azote et analysés.

Les analyses chromatographiques des insaponifiables obtenus ont donné les résultats suivants (% en masse) :

| N° essai | % eau ajoutée après séchage | Température de pression | Rendement d'extraction de l'huile | Taux de distillation | Rendement en insaponifiable | Composition de l'insaponifiable | |
|---|---|---|---|---|---|---|---|
| | | | | | | Teneur en stérols | Teneur en Hydrocarbures Aliphatiques saturés |
| | % | °C | % | % | % | m/m | m/m |
| 1 | 0 | 80 | 15.2 | 11,09 | 58,4 | 7,63 | 0,40 |
| 2 | 3 | 80 | 40.0 | 11,40 | 58,3 | 7,90 | 0,42 |
| 3 | 3 | 100 | 40.4 | 12,40 | 59,9 | 8,40 | 0,58 |

Les essais n°1 et 2 mettent en évidence que l'addition d'eau aux fruits séchés avant pression permet d'atteindre un rendement de pression élevé dans le cas de l'essai n°2, contrairement à l'essai n°1 dans lequel aucune opération d'addition d'eau n'a été mise en œuvre.

Les essais 2 et 3, comparativement à l'essai 1, mettent en évidence le bénéfice cumulé de la température et de l'addition d'eau aux avocats secs sur le rendement de pression et sur la composition de l'insaponifiable, notamment sur sa teneur en stérols et hydrocarbures aliphatiques saturés.

Exemple 2 comparatif: pulpe seule

100 kg de pulpe fraiche, mise en purée, sont étalés sur des plateaux en fines couches de quelques millimètres et le séchage est opéré en étuve ventilée à une température de 80°C pendant 24 heures. Le produit séché est ensuite broyé dans un broyeur à marteaux.

29,5 kg de pulpe sèche sont récupérés représentant une perte au séchage de 70,5%.

L'huile est extraite par pression mécanique sur une presse de laboratoire du type Komet après addition de 2% d'eau sur la pulpe séchée et homogénéisation ½ heure dans un mélangeur planétaire. L'huile est ensuite filtrée sur filtre cloche sous pression d'azote et conditionnée sous atmosphère d'azote.

L'huile filtrée est alors distillée sur un distillateur moléculaire à film raclé du type Leybold KDL 4 à une température de 230°C et sous un vide de $10^{-2}$ à $10^{-3}$ mmHg. Le taux de distillation obtenu pour cette opération est de 10,8%.

Dans l'étape suivante, le distillat concentré en insaponifiable est chauffé à 85°C pendant 48h sous courant d'azote dans un réacteur agitée.

Le distillat en solution dans l'alcool éthylique est ensuite saponifié par de la potasse à 50% et chauffage à reflux de l'alcool pendant 4 heures.

Le mélange réactionnel est alors dilué par de l'eau purifiée puis extrait avec du 1,2-dichloroéthane dans une ampoule à décantée. L'extraction est poursuivie avec des volumes répétés de solvant jusqu'à obtention d'une phase organique inférieure incolore.

Les phases organiques sont regroupées, lavées à l'eau jusqu'à pH neutre, séchées sur sulfate de sodium anhydre puis évaporées sous vide dans un évaporateur rotatif pour éliminer le solvant et récupérer l'insaponifiable. Le produit récupéré est alors conditionné sous azote et analysé.

Les analyses chromatographiques de l'insaponifiable obtenu ont donné les résultats suivants (% en masse) :

Teneur en alkyls furanes : 67,9% ;
Teneur en alkyls polyols : 23,5% ;
Teneur en stérols : 8,5% ;
Teneur en hydrocarbures aliphatiques saturés : 0,1%.

Le protocole opératoire décrit dans l'exemple 2 a été reproduit ensuite dans les exemples suivants présentés ci-dessous. Les pourcentages sont exprimés en masse.

Exemple 3 : mélange de pulpe avec de la peau à 20%

80 kg de pulpe humide, sont broyés avec 20 kg de peaux humides, puis étalés sur des plateaux en un film mince de quelques millimètres d'épaisseur.

28,8 kg de pulpe sèche + peau sont récupérés représentant une perte au séchage de 71,2 %.

Les analyses chromatographiques de l'insaponifiable final obtenu ont donné les résultats suivants :

Teneur en alkyls furanes : 67,2% ;
Teneur en alkyls polyols : 23,2% ;
Teneur en stérols : 9,1% ;
Teneur en hydrocarbures aliphatiques saturés : 0,5%.

Ainsi, l'utilisation de 20% de peau dans le mélange d'avocat mis en œuvre permet de multiplier par 5 la teneur en hydrocarbures aliphatiques saturés et d'augmenter de 7% la teneur en stérols dans l'insaponifiable préparé par rapport à un produit préparé à partir de pulpe seule (exemple 2).

Exemple 4 : mélange de pulpe avec de la peau à 40%

60 kg de pulpe humide sont broyés avec 40 kg de peaux humides, puis étalés sur des plateaux en un film mince de quelques millimètres d'épaisseur.

28,1 kg de pulpe sèche + peau sont récupérés représentant une perte au séchage de 71,9 %.

Les analyses chromatographiques de l'insaponifiable final obtenu ont donné les résultats suivants :

Teneur en alkyls furanes : 65,9% ;
Teneur en alkyls polyols : 22,8% ;
Teneur en stérols : 9,8% ;
Teneur en hydrocarbures aliphatiques saturés : 1,5%.

Ainsi, l'utilisation de 40% de peau dans le mélange d'avocat mis en œuvre permet de multiplier par 15 la teneur en hydrocarbures aliphatiques saturés et d'augmenter de 15% la teneur en stérols dans l'insaponifiable préparé par rapport à un produit préparé à partir de pulpe seule (exemple 2).

Exemple 5 : Fruits durs entiers dont peau 15%

La force de résistance à la pénétration moyenne des avocats est supérieure à 13kg/cm$^2$.

100 kg de fruits entiers frais sont tranchés en lamelles de 2 à 5 mm, puis répartis en couches régulières sur des plateaux.

31,2 kg d'avocats séchés sont récupérés représentant une perte au séchage de 68,8 %.

Les analyses chromatographiques de l'insaponifiable final obtenu ont donné les résultats suivants :

Teneur en alkyls furanes : 67,0% ;
Teneur en alkyls polyols : 23,6% ;
Teneur en stérols : 8,8% ;
Teneur en hydrocarbures aliphatiques saturés : 0,58%.

Ainsi, l'utilisation de 15% de peau dans le mélange d'avocat mis en œuvre permet de multiplier par 5,8 la teneur en hydrocarbures aliphatiques saturés et d'augmenter de 3,5% la teneur en stérols dans l'insaponifiable préparé par rapport à un produit préparé à partir de pulpe seule (exemple 2).

Exemple 6 : Fruits durs entiers auxquels on ajoute de la peau (32% de peau)

La peau est présente ici à une teneur de 32% en masse dans le mélange de départ mis en œuvre (fruits durs entiers + peau).

La force de résistance à la pénétration moyenne des avocats est supérieure à 13kg/cm$^2$.

80 kg de fruits durs entiers frais auxquels sont ajoutés 20 kg de peaux humides sont tranchés en lamelles de 2 à 5 mm, puis répartis en couches régulières sur des plateaux.

37,7 kg d'avocats séchés + peau sont récupérés représentant une perte au séchage de 62,3 %.

Les analyses chromatographiques de l'insaponifiable final obtenu ont donné les résultats suivants :

Teneur en alkyls furanes : 66,0% ;
Teneur en alkyls polyols : 23,2% ;
Teneur en stérols : 9,6% ;
Teneur en hydrocarbures aliphatiques saturés : 1,18%.

Ainsi, l'utilisation de 32% de peau dans le mélange d'avocat mis en œuvre permet de multiplier par 11,8 la teneur en hydrocarbures aliphatiques saturés et d'augmenter de 13% la teneur en stérols dans l'insaponifiable préparé par rapport à un produit préparé à partir de pulpe seule (exemple 2).

Exemple 7 : Fruits durs entiers auxquels on ajoute de la peau (49% de peau)

La peau est présente ici à une teneur de 49% en masse dans le mélange de départ mis en œuvre (fruits durs entiers + peau).

La force de résistance à la pénétration moyenne des avocats est supérieure à 13kg/cm$^2$.

60 kg de fruits entiers frais auxquels sont ajoutés 40 kg de peaux humides sont tranchés en lamelles de 2 à 5 mm, puis répartis en couches régulières sur des plateaux.

44,2 kg d'avocats séchés + peau sont récupérés représentant une perte au séchage de 55,8 %.

Les analyses chromatographiques de l'insaponifiable final obtenu ont donné les résultats suivants :

Teneur en alkyls furanes : 65,1%;
Teneur en alkyls polyols : 23,0% ;
Teneur en stérols : 10,1% ;
Teneur en hydrocarbures aliphatiques saturés : 1,79%.

Ainsi, l'utilisation de 49% de peau dans le mélange d'avocat mis en œuvre permet de multiplier par 17,9 la teneur en hydrocarbures aliphatiques saturés et d'augmenter de 18,8% la teneur en stérols dans l'insaponifiable préparé par rapport à un produit préparé à partir de pulpe seule (exemple 2).

Exemple 8 comparatif : Fruits mous entiers dont peau (15%)

La force de résistance à la pénétration moyenne des avocats est inférieure à 2kg/cm$^2$.

100 kg d'avocat mous entiers sont broyés dans un broyeur à couteaux, puis étalés sur des plateaux en un film mince de quelques millimètres d'épaisseur.

35,2 kg d'avocats séchés sont récupérés représentant une perte au séchage de 64,8 %.

Les analyses chromatographiques de l'insaponifiable final obtenu ont donné les résultats suivants :

Teneur en alkyls furanes : 62,4% ;
Teneur en alkyls polyols : 24,9% ;
Teneur en stérols : 9,2% ;
Teneur en hydrocarbures aliphatiques saturés : 0,52%.

Ainsi, l'utilisation de 15% de peau dans le mélange d'avocat mou mis en œuvre permet de multiplier par 5,2 la teneur en hydrocarbures aliphatiques saturés et d'augmenter de 8,2% la teneur en stérols dans l'insaponifiable préparé par rapport à un produit préparé à partir de pulpe seule (exemple 2).

Le tableau ci-dessous synthétise les résultats des différents essais 2 à 8 :

| N° essai | Matière extraite d'avocat | Teneur en stérols | Augmentation en stérols par rapport à pulpe seule | Teneur en hydrocarbures aliphatiques saturés | Augmentation en hydrocarbures par rapport à pulpe seule |
|---|---|---|---|---|---|
| | | % m/m | % | % m/m | % |
| 2 | Pulpe | 8,5 | - | 0,10 | - |
| 3 | 80% Pulpe 20% peau | 9,1 | 7 | 0,50 | 500 |
| 4 | 60% Pulpe 40% peau | 9,8 | 15 | 1,50 | 1500 |
| 5 | Avocats durs entiers dont peau 15% | 8,8 | 3,5 | 0,58 | 580 |
| 6 | Avocats durs entiers dont peau 32% | 9,6 | 13 | 1,18 | 1180 |
| 7 | Avocats durs entiers dont peau 49% | 10,1 | 18,8 | 1,79 | 1790 |
| 8 | Avocats mous entiers dont peau 15% | 9,2 | 8,2 | 0,52 | 520 |

## Revendications

1.  Procédé d'obtention d'un insaponifiable d'avocat enrichi en hydrocarbures aliphatiques saturés et en stérols à partir d'au moins de la peau d'avocat, ledit insaponifiable contenant au moins 0,2% en masse d'hydrocarbures aliphatiques saturés et au moins 1% en masse de stérols, par rapport à la masse totale de l'insaponifiable, ladite peau d'avocat représentant 5 à 50% en masse par rapport à la masse totale d'avocats utilisés, lesdits avocats utilisés n'étant pas des avocats mous ayant une force de résistance à la pénétration dans la chair inférieure ou égale à 3 kg/cm$^2$, ladite force de résistance étant mesurée à l'aide d'un pénétromètre, et ledit procédé comprenant les étapes successives suivantes :

    (1) Tranchage ou broyage d'avocats contenant une teneur en peau de 5 à 50% m/m, ledit broyage conduisant à l'obtention d'une granulométrie de broyat comprise entre 2 et 20 mm,
    (2) Séchage à haute température comprise entre 60 et 150°C, jusqu'à l'obtention d'une humidité résiduelle inférieure ou égale à 5%,
    (3) Addition d'eau aux avocats séchés par ajout de 1 à 5 % d'eau ou de vapeur d'eau par rapport à la masse des avocats secs,
    (4) Extraction de l'huile par pression mécanique, puis
    (5) alternativement :

       - a. Traitement thermique de l'huile extraite à une température comprise entre 80 et 150°C, puis concentration de l'huile en sa fraction insaponifiable, ou bien

- b. Concentration de l'huile en sa fraction insaponifiable, puis traitement thermique à une température comprise entre 80 et 150°C,

(6) suivie d'une étape de saponification et d'extraction de l'insaponifiable,
(7) et éventuellement au moins une purification et/ou un fractionnement.

2. Procédé d'obtention d'un insaponifiable d'avocat enrichi en hydrocarbures aliphatiques saturés et en stérols selon la revendication 1, **caractérisé en ce que** le séchage (2) est réalisé à une température comprise entre 80 et 120°C

3. Procédé d'obtention d'un insaponifiable d'avocat enrichi en hydrocarbures aliphatiques saturés et en stérols selon la revendication 1, **caractérisé en ce que** l'extraction de l'huile (4) par pression mécanique est réalisée à une température comprise entre 80 et 100°C.

4. Procédé d'obtention d'un insaponifiable d'avocat enrichi en hydrocarbures aliphatiques saturés et en stérols selon l'une quelconques des revendications 1 à 3, **caractérisé en ce que** la peau d'avocat représente 10 à 40% en masse par rapport à la masse totale d'avocats utilisés.

5. Insaponifiable d'avocat enrichi en hydrocarbures aliphatiques saturés et en stérols, susceptible d'être obtenu par le procédé selon l'une quelconque des revendications 1 à 4, **caractérisé en ce qu'**il contient au moins 0,2% en masse d'hydrocarbures aliphatiques saturés et au moins 1% en masse de stérols, par rapport à la masse totale de l'insaponifiable.

6. Insaponifiable d'avocat enrichi en hydrocarbures aliphatiques saturés et en stérols selon la revendication 5, **caractérisé en ce qu'**il contient entre 0,2 et 10% en masse d'hydrocarbures aliphatiques saturés, par rapport à la masse totale de l'insaponifiable.

7. Insaponifiable d'avocat enrichi en hydrocarbures aliphatiques saturés et en stérols selon la revendication 5 ou 6, **caractérisé en ce que** les hydrocarbures aliphatiques saturés sont des hydrocarbures à chaîne linéaire non ramifiée.

8. Insaponifiable d'avocat enrichi en hydrocarbures aliphatiques saturés et en stérols selon la revendication 7, **caractérisé en ce que** les hydrocarbures aliphatiques saturés sont choisis dans le groupe constitué par le n-heptacosane, le n-nonacosane, le n-hentriacontane, et leurs mélanges.

9. Insaponifiable d'avocat enrichi en hydrocarbures aliphatiques saturés et en stérols selon l'une quelconques des revendications 5 à 8, **caractérisé en ce qu'**il contient entre 1 et 20% en masse, de stérols, par rapport à la masse totale de l'insaponifiable.

10. Insaponifiable d'avocat enrichi en hydrocarbures aliphatiques saturés et en stérols selon l'une quelconque des revendications 5 à 9, **caractérisé en ce que** les stérols sont choisis dans le groupe constitué par le β-Sitostérol, le Campestérol, le Stigmastérol, le Δ5-Avenastérol, le Δ7-Stigmastérol, le Citrostadiénol, et leurs mélanges.

11. Insaponifiable d'avocat enrichi en hydrocarbures aliphatiques saturés et en stérols selon l'une quelconque des revendications 5 à 10, **caractérisé en ce qu'**il contient des alkyls polyols et/ou des alkyls furanes.

12. Insaponifiable d'avocat enrichi en hydrocarbures aliphatiques saturés et en stérols selon l'une quelconque des revendications 5 à 11, pour son utilisation comme médicament.

13. Insaponifiable d'avocat enrichi en hydrocarbures aliphatiques saturés et en stérols selon la revendication 12, pour son utilisation dans la prévention et/ou le traitement des troubles du tissu conjonctif tels que l'arthrose, des pathologies articulaires telles que les rhumatismes, ou encore des maladies parodontales, telles que la gingivite ou la parodontite.


**Patentansprüche**

1. Verfahren zur Herstellung eines mit gesättigten aliphatischen Kohlenwasserstoffen und mit Sterolen angereicherten Avocado-Unverseifbaren aus mindestens der Avocadoschale, wobei das Unverseifbare mindestens 0,2 Ma% gesättigte aliphatische Kohlenwasserstoffe und mindestens 1 Ma% Sterole in Bezug auf die Gesamtmasse des Unverseifbaren enthält, wobei die Avocadoschale 5 bis 50 Ma% in Bezug auf die Gesamtmasse verwendeter Avocados darstellt, wobei die verwendeten Avocados keine weichen Avocados sind mit einer Widerstandskraft gegenüber Eindringen in das Fleisch von unter oder gleich 3 kg/cm$^2$ aufweist, wobei die Widerstandskraft mit Hilfe eines Penetrometers gemessen wird, wobei das Verfahren die folgenden aufeinanderfolgenden Schritte umfasst:

(1) Schneiden oder Zerkleinern von Avocados mit einem Gehalt an Schale von 5 bis 50% m/m, wobei das Zerkleinern zu einer Mahlgut-Teilchengröße zwischen 2 und 20 mm führt,
(2) Trocknen bei hoher Temperatur zwischen 60 und 150°C bis zum Erhalt einer Restfeuchte von unter oder gleich 5%,
(3) Hinzufügen von Wasser zu den getrockneten Avocados durch Hinzugabe von 1 bis 5% Wasser oder Wasserdampf in Bezug auf die Masse der trockenen Avocados,
(4) Extrahieren des Öls durch mechanisches Pressen, dann
(5) alternativ:

- a. thermisches Behandeln des extrahierten Öls bei einer Temperatur zwischen 80 und 150°C, dann Konzentrieren des Öls in seiner unverseifbaren Fraktion, oder auch
- b. Konzentrieren des Öls in seiner unverseifbaren Fraktion, dann thermisches Behandeln bei einer Temperatur zwischen 80 und 150°C,

(6) gefolgt von einem Schritt der Verseifung und der Extraktion des Unverseifbaren,
(7) und eventuell mindestens einer Reinigung und/oder einer Fraktionierung.

**2.** Verfahren zur Herstellung eines mit gesättigten aliphatischen Kohlenwasserstoffen und mit Sterolen angereicherten Avocado-Unverseifbaren nach Anspruch 1, **dadurch gekennzeichnet, dass** das Trocknen (2) bei einer Temperatur zwischen 80 und 120°C durchgeführt wird.

**3.** Verfahren zur Herstellung eines mit gesättigten aliphatischen Kohlenwasserstoffen und mit Sterolen angereicherten Avocado-Unverseifbaren nach Anspruch 1, **dadurch gekennzeichnet, dass** die Extraktion des Öls (4) durch mechanisches Pressen bei einer Temperatur zwischen 80 und 100°C durchgeführt wird.

**4.** Verfahren zur Herstellung eines mit gesättigten aliphatischen Kohlenwasserstoffen und mit Sterolen angereicherten Avocado-Unverseifbaren nach einem der Ansprüche 1 bis 3, **dadurch gekennzeichnet, dass** die Avocadoschale 10 bis 40 Ma% in Bezug auf die Gesamtmasse verwendeter Avocados darstellt.

**5.** Avocado-Unverseifbares, angereichert mit gesättigten aliphatischen Kohlenwasserstoffen und mit Sterolen, herstellbar durch das Verfahren nach einem der Ansprüche 1 bis 4, **dadurch gekennzeichnet, dass** es mindestens 0,2 Ma% gesättigte aliphatische Kohlenwasserstoffe und mindestens 1 Ma% Sterole in Bezug auf die Gesamtmasse des Unverseifbaren enthält.

**6.** Avocado-Unverseifbares, angereichert mit gesättigten aliphatischen Kohlenwasserstoffen und mit Sterolen nach Anspruch 5, **dadurch gekennzeichnet, dass** es zwischen 0,2 und 10 Ma% gesättigte aliphatische Kohlenwasserstoffe in Bezug auf die Gesamtmasse des Unverseifbaren enthält.

**7.** Avocado-Unverseifbares, angereichert mit gesättigten aliphatischen Kohlenwasserstoffen und mit Sterolen nach Anspruch 5 oder 6, **dadurch gekennzeichnet, dass** die gesättigten aliphatischen Kohlenwasserstoffe Kohlenwasserstoffe mit nicht verzweigter linearer Kette sind.

**8.** Avocado-Unverseifbares, angereichert mit gesättigten aliphatischen Kohlenwasserstoffen und mit Sterolen nach Anspruch 7, **dadurch gekennzeichnet, dass** die gesättigten aliphatischen Kohlenwasserstoffe aus der Gruppe ausgewählt sind, die von dem n-Heptacosan, dem n-Nonacosan, dem n-Hentriacontan und deren Gemischen gebildet ist.

**9.** Avocado-Unverseifbares, angereichert mit gesättigten aliphatischen Kohlenwasserstoffen und mit Sterolen nach einem der Ansprüche 5 bis 8, **dadurch gekennzeichnet, dass** es zwischen 1 und 20 Ma% Sterole in Bezug auf die Gesamtmasse des Unverseifbaren enthält.

**10.** Avocado-Unverseifbares, angereichert mit gesättigten aliphatischen Kohlenwasserstoffen und mit Sterolen nach einem der Ansprüche 5 bis 9, **dadurch gekennzeichnet, dass** die Sterole aus der Gruppe ausgewählt sind, die von dem β-Sitosterol, dem Campesterol, dem Stigmasterol, dem Δ5-Avenasterol, dem Δ7-Stigmasterol, dem Citrostadienol und deren Gemischen gebildet ist.

11. Avocado-Unverseifbares, angereichert mit gesättigten aliphatischen Kohlenwasserstoffen und mit Sterolen nach einem der Ansprüche 5 bis 10, **dadurch gekennzeichnet, dass** es Alkylpolyole und/oder Alkylfurane enthält.

12. Avocado-Unverseifbares, angereichert mit gesättigten aliphatischen Kohlenwasserstoffen und mit Sterolen nach einem der Ansprüche 5 bis 11 für seine Verwendung als Arzneimittel.

13. Avocado-Unverseifbares, angereichert mit gesättigten aliphatischen Kohlenwasserstoffen und mit Sterolen nach Anspruch 12 für seine Verwendung zur Vorbeugung und/oder Behandlung von Erkrankungen des Bindegewebes wie der Arthrose, der Gelenkerkrankungen wie Rheuma oder auch von Parodontalkrankheiten wie der Gingivitis oder der Parodontitis.

**Claims**

1. A method for obtaining avocado unsaponifiable enriched in saturated aliphatic hydrocarbons and in sterols from at least avocado peels, said unsaponifiable containing at least 0.2% saturated aliphatic hydrocarbons by weight and at least 1% sterols by weight, relative to the total weight of the unsaponifiable, said avocado peels accounting for 5 to 50% by weight relative to the total weight of avocados used, said avocados used not being soft avocados having a pulp penetration resistance of less than or equal to 3 kg/cm$^2$, said resistance being measured using a penetrometer, and said method comprising the following successive steps:

   (1) Cutting or grinding of avocados containing a peel content of 5 to 50% w/w, said grinding producing a particle size distribution of the ground material between 2 and 20 mm,
   (2) High-temperature drying at a temperature between 60 and 150°C, until a residual moisture content less than or equal to 5% is obtained,
   (3) Addition of water to the dried avocados by adding 1 to 5% water or water vapor relative to the weight of dried avocados,
   (4) Oil extraction by mechanical pressure, and then
   (5) alternatively:

      - a. Heat treatment of the extracted oil at a temperature between 80 and 150°C, and then concentration of the oil in its unsaponifiable fraction, or
      - b. Concentration of the oil in its unsaponifiable fraction, and then heat treatment at a temperature between 80 and 150°C,

   (6) followed by a step of saponification and extraction of the unsaponifiable,
   (7) and optionally at least a purification and/or fractionation.

2. The method for obtaining avocado unsaponifiable enriched in saturated aliphatic hydrocarbons and in sterols according to claim 1, **characterized in that** drying (2) is carried out at a temperature between 80 and 120°C.

3. The method for obtaining avocado unsaponifiable enriched in saturated aliphatic hydrocarbons and in sterols according to claim 1, **characterized in that** oil extraction (4) by mechanical pressure is carried out at a temperature between 80 and 100°C.

4. The method for obtaining avocado unsaponifiable enriched in saturated aliphatic hydrocarbons and in sterols according to any one of claims 1 to 3, **characterized in that** the avocado peels account for 10 to 40% by weight relative to the total weight of avocados used.

5. Avocado unsaponifiable enriched in saturated aliphatic hydrocarbons and in sterols, obtainable by the method according to any one of claims 1 to 4, **characterized in that** it contains at least 0.2% saturated aliphatic hydrocarbons by weight and at least 1% sterols by weight, relative to the total weight of the unsaponifiable.

6. Avocado unsaponifiable enriched in saturated aliphatic hydrocarbons and in sterols according to claim 5, **characterized in that** it contains between 0.2 and 10% saturated aliphatic hydrocarbons by weight, relative to the total weight of the unsaponifiable.

7. Avocado unsaponifiable enriched in saturated aliphatic hydrocarbons and in sterols according to claim 5 or 6, **characterized in that** the saturated aliphatic hydrocarbons are linear unbranched hydrocarbons.

8. Avocado unsaponifiable enriched in saturated aliphatic hydrocarbons and in sterols according to claim 7, **characterized in that** the saturated aliphatic hydrocarbons are selected from the group consisting of n-heptacosane, n-nonacosane, n-hentriacontane, and mixtures thereof.

9. Avocado unsaponifiable enriched in saturated aliphatic hydrocarbons and in sterols according to any one of claims 5 to 8, **characterized in that** it contains between 1 and 20% sterols by weight, relative to the total weight of the unsaponifiable.

10. Avocado unsaponifiable enriched in saturated aliphatic hydrocarbons and in sterols according to any one of claims 5 to 9, **characterized in that** the sterols are selected from the group consisting of β-sitosterol, campesterol, stigmasterol, Δ5-avenasterol, Δ7-stigmasterol, citrostadienol, and mixtures thereof.

11. Avocado unsaponifiable enriched in saturated aliphatic hydrocarbons and in sterols according to any one of claims 5 to 10, **characterized in that** it contains alkyl polyols and/or alkyl furans.

12. Unsaponifiable avocado enriched in saturated aliphatic hydrocarbons and in sterols according to any one of claims 5 to 11, for use as a drug.

13. Avocado unsaponifiable enriched in saturated aliphatic hydrocarbons and in sterols according to claim 12, for use in the prevention and/or treatment of conjunctive tissue disorders such as arthrosis, articular pathologies such as rheumatism, or periodontal diseases such as gingivitis or periodontitis.

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2013098293 A

**Littérature non-brevet citée dans la description**

- Extraction, characterization, and prediction of the oxidative stability of avocado oil. **BIZIMANA V.** Dissertation. University of Minnesota, 1997, 1-241